# EUROPEAN PATENT APPLICATION

(11) **EP 1 420 021 A1**
(43) Date of publication of application: **19.05.2004**
(21) Application number: 04003183.3
(22) Date of filing: 25.05.2000
(51) Int. Cl.: C07D 473/00, C07D 473/34, C07H 19/16, C07H 19/20, A61K 31/52, A61P 31/00

(54) **DNA Methyltransferase inhibitors**

(30) Priority: 25.05.1999 US 135870 P; 17.09.1999 US 154582 P; 03.01.2000 US 174256 P
(62) Divisional of application: 00964879.1
(71) Applicant: The Penn State Research Foundation, State College, PA 16802 (US)
(72) Inventor: The designation of the inventor has not yet been filed
(74) Representative: Grünecker, Kinkeldey, Stockmair & Schwanhäusser Anwaltssozietät

(57) **Abstract**

A compound of the formula 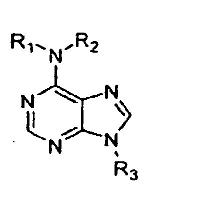 or a pharmaceutically acceptable salt thereof,
- wherein: R¹, R², and R³ are the same or different and are independently hydrogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from oxygen and nitrogen, and where any member of the alkyl, aryl or cycloalkyl group is optionally substituted with halogen, lower alkyl or lower alkoxy, aryl or substituted aryl, and
- where: R³ can be ribose, deoxyribose or phosphorylated derivatives thereof,
- wherein: R¹, R², and R³ are not all hydrogen and
- wherein: when R³ is ribose, deoxyribose or phosphorylated derivatives thereof, one of R¹ or R² is not hydrogen.

## Description

### BACKGROUND OF THE INVENTION

### 1. Field of the Invention

The present invention relates to the field of antibiotics and particularly antibacterial compounds. The invention specifically relates to antibiotics targeted to DNA modification enzymes, in particular adenine DNA methyltransferases, that are the components of a broad variety of different bacterial pathogens including those that are essential for bacterial cell growth. The invention particularly provides inhibitors of such adenine DNA methyltransferases having little or no inhibitory effects on cytosine methyltransferases, and hence having limited antibiotic effect on eukaryotic, particularly mammalian, cells. Methods for preparing and using the adenine DNA methyltransferase inhibitors of the invention, and pharmaceutical compositions thereof, are also provided.

### 2. Background of the Invention

One hallmark of the modern era of medicine has been the decline in morbidity and mortality associated with bacterial infections. The development of a variety of antibiotic drugs in the early and middle parts of the twentieth century provided medical practitioners for the first time with effective treatments for a variety of infectious diseases.

However, misuse of conventional antibiotics and natural selection of the infectious bacterial population has resulted in the development of varying degrees of drug resistance by most bacterial infectious agents to most antibiotic agents. In severe cases, such as MRSA (Multidrug-Resistant StaphA), one or only a few antibiotics are currently effective. In addition, the existence of immunodeficiency syndromes results in additional incidence of opportunistic infections requiring intensive antibiotic treatment.

Thus, there is an increasing need in the art for novel, more effective antibiotic compounds for treating bacterial infections that are resistant to currently available therapies.

Most bacteria modify their genomic DNA by methylation of specific nucleotide bases. DNA methylation is critical to gene regulation and repair of mutational lesions (see Jost & Soluz, 1993, DNA METHYLATION, MOLECULAR BIOLOGY AND BIOLOGICAL SIGNIFICANCE, Birhauser Verlag: Basel, Switzerland; Palmer & Marinus, 1994, *Gene* 143: 1-12; Dryden, 1999, "Bacterial DNA Methyltransferases," in S-ADENOSYLMETHIONINE-DEPENDENT METHYLTRANSFERASES: STRUCTURES AND FUNCTIONS, X. Cheng and R. M. Blumenthal (eds.), World Scientific Publishing, p.283-340 *for review*). DNA methylation is catalyzed by a class of enzymes having different sequences specificities. There are those DNA methyltransferases for example (*dam*) that methylate adenine residues in GATC sequences or cytosine (*dcm*) residues in CCAGG or CCTGG sequences which are not contained in the recognition site of a cognate restriction enzyme. There are those DNA methyltransferases that methylate residues contained in the recongnition site of a cognate restriction enzyme (*for example, Apa*I, *Ava*II, *Bcl*I*, Cla*I, *Dpn*II, *Eco*RI*, Hae*III, *Hha*I*, Mbo*I, and *Msp*I; *see*, Marinus & Morris, 1973, *J. Bacteriol.* 114: 1143-1150; May & Hatman, 1975, *J. Bacreriol.* 123: 768-770; Heitman, 1993, *Genet. Eng.* 15: 57-108). In addition, the instant inventors have discovered an adenine DNA methyltransferase from *Caulobacter cresentus* that methylatcs the adenine residue in the sequence GANTC, as disclosed in International Application Publication No. WO98/12206. This methyltransferase is cell-cycle regulated and essential for successful bacterial cell growth; inhibition of the enzyme makes the bacteria non-viable. Similar methyltransferases have also been discovered in *Brucella abortus, Helicobacter pylori, Agrobacterium tumefaciens* and *Rhizobium meliloti.* In contrast with bacterial cells, DNA methylation in eukaryotic, and particularly mammalian cells, is limited to cytosine methylation at sites comprising the sequence CpG (Razin & Riggs, 1980, *Science* 210: 604-610; Jost & Bruhat, 1997, *Prog. Nucleic Acid Res. Molec. Biol.* 57: 217-248).

Thus, the existence of DNA methylation, in particular, the cell-cycle regulated adenine DNA methyltransferase found by the inventors in certain bacterial species, addresses the need in the art for novel targets for antibiotic activity.

### SUMMARY OF THE INVENTION

The invention provides antibiotic compounds capable of inhibiting adenine DNA methyltransferases in bacterial cells. The antibiotic compounds of the invention specifically inhibit adenine-specific bacterial DNA methyltransferases, and do not inhibit bacterial or eukaryotic, particularly mammalian and most particularly human, cytosine-specific DNA methyltransferases. The compounds of the invention also inhibit adenine-specific DNA methyltransferases in plants. The antibiotic compounds are also provided as pharmaceutical compositions capable of being administered to an animal, most preferably a human, for treatment of a disease having a bacterial etiology, or an opportunistic infection with a bacteria in an animal, most preferably a human, in an immunologically compromised or debilitated state of health.

The invention also provides methods for preparing the antibiotic compounds and pharmaceutical compositions thereof, and methods of using said antibiotics therapeutically. Kits and packaged embodiments of the antibiotic compounds and pharmaceutical compositions of the invention are also provided.

Specific preferred embodiments of the present invention will become evident from the following more detailed description of certain preferred embodiments and the claims.

### DESCRIPTION OF THE DRAWINGS

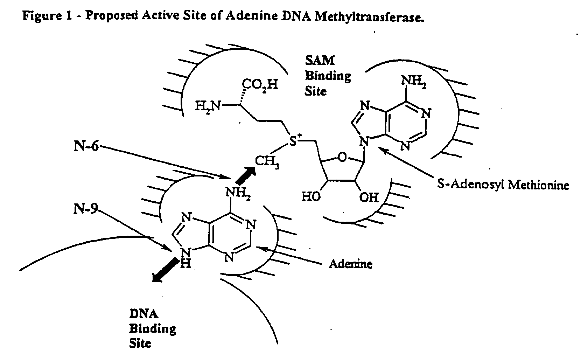

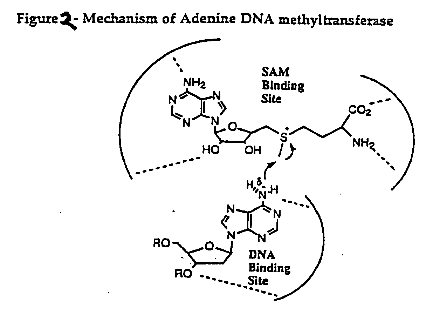

Figures 1 and 2 depict schematic diagrams of the "active site" of bacterial adenine DNA methyltransferases.

### DETAILED DESCRIPTION OF THE PREFERRED EMBODIMENTS

This invention provides antibiotics, and specifically antibacterial compounds, that are inhibitors of bacterial adenine DNA methyltransferases. The compounds of the invention exhibit antibacterial, growth-inhibitory properties against any bacterial species that produces an adenine DNA methyltransferase. These include adenine DNA methyltransferases that are components of bacterial restriction/modification systems as understood in the art, as well as cell-cycle regulated adenine DNA methyltransferases (CcrM), such as those disclosed in International Application Publication No. WO98/12206, incorporated by reference. Thus, inhibitors of adenine DNA methyl transferases are particularly provided by the invention.

The adenine DNA methyltransferase inhibitors of the invention comprise a novel class of broad-spectrum antibiotics. Most bacterial species possess a DNA methyltransferase that is part of a modification apparatus, typically associated with a restriction enzyme, that preserves the integrity of cellular DNA while providing a defense against foreign (most typically viral) DNA. In addition, certain bacteria produce an adenine DNA methyltransferase that is essential for bacterial cell growth. Medically-important bacterial species that provide appropriate targets for the antibacterial activity of the inhibitors of the invention include gram-positive bacteria, including cocci such as *Staphylococcus* species and *Streptococcus* species; bacilli, including *Bacillus* species, *Corynebacterium* species and *Clostridium* species; filamentous bacteria, including *Actinomyces* species and *Streptomyces* species; gram-negative bacteria, including cocci such as *Neisseria* species; bacilli, such as *Pseudomonas* species, *Brucella* species, *Agrobacterium* species, *Bordetella* species, *Escherichia* species, *Shigella* species, *Yersinia* species, *Salmonella* species, *Klebsiella* species, *Enterobacter* species, *Hemophilus* species, *Pasteurella* species, and *Streptobacillus* species; spirochetal species, *Campylobacter* species, *Vibrio* species; and intracellular bacteria including *Rickettsiae* species and *Chlamydia* species.

Specific bacterial species that are targets for the adenine DNA methyltransferase inhibitors of the invention include *Staphylococcus aureus; Staphylococcus saprophyticus; Streptococcus pyrogenes; Streptococcus agalactiae; Streptococcus pneumoniae; Bacillus anthracis; Corynebacterium diphtheria; Clostridium perfringens; Clostridium botulinum; Clostridium tetani; Neisseria gonorrhoeae; Neisseria meningitidis; Pseudomonas aeruginosa; Legionella pneumophila; Escherichia coli; Yersinia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus* and *Agrobacterium tumefaciens.*

It is an important property of the adenine DNA methyltransferase inhibitors of the invention that the level of activity of these substances with cytosine-specific DNA methyltransferases is low. This is because cytosine-specific DNA methyltransferases occur in mammalian, most particularly human, cells, and it is an advantageous property of the adenine DNA methyltransferases of the invention to have little or no inhibitory activity against mammalian methyltransferases. This property confers upon the molecules provided by the invention the beneficial property of being bacterial cell specific, and having little antibiotic activity against mammalian, most preferably human, cells. Preferably, the IC₅₀ of these compounds for cytosine-specific DNA methyltransferases is greater than 500µM.

The inhibitory compounds provided by the invention are represented by Formula I: 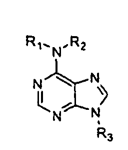 where R¹ , R² and R³ are the same or different and are independently hydrogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from oxygen and nitrogen, and where any member of the alkyl, aryl or cycloalkyl group is optionally substituted with halogen, lower alkyl or lower alkoxy, aryl or substituted aryl, and where R³ can be ribose, deoxyribose or phosphorylated derivatives thereof, including phosphorothioates, phosphoramidites and similar derivatives known in the art, provided that R¹ , R² and R³ are not all hydrogen, and where R³ is ribose, deoxyribose or phosphorylated derivatives thereof, R¹ and R² are not both hydrogen. In preferred embodiments, R¹ is H, R¹ is (2-diphenylborinic ester) ethyl or diphenylpropyl, and R³ is H, 2-(4-morpholinyl)-ethyl, 3-(N-phthaloyl)-aminopropyl, 2-(2-(2-hydroxyethoxy)ethoxy)ethyl, or ethyl-2-(acrylate)-methyl. In additional preferred embodiments, R¹ is H, R² is (S-homocysteinyl)methyl and R³ is ribose, 5' phosphorylribose, deoxyribose or 5' phosphoryl deoxyribose. In other preferred embodiments, R³ is H and R¹ and R² are together 2-(diphenylmethyl) cyclopentyl or 2-(diphenylhydroxymethyl) cyclopentyl. In further preferred embodiments, R¹ is H, R² is alanylbutyl ester, 2-carboximido-2-aminoethyl, 2-aminoethyl or mono- or bisubstituted 2-amino ethyl, and R³ is 2-(4-morpholinyl)-ethyl.

The invention also provides compounds of Formula II: 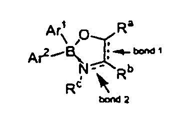 wherein bonds 1 and 2 can be double or single, Ar¹ and Ar² can be the same or different and are each independently aryl or heteroaryl, or aryl or heteroaryl substituted at one or a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, and R^{a}, R^{b} and R^{c} are the same or different and are independently hydrogen, halogen, nitro, nitroso, lower alkyl, lower alkoxy, lower alkoxyalkyl, or cycloalkyl or cycloalkyl alkoxy, or aryl or heteroaryl, or aryl or heteroaryl substituted at one or a plurality of positions with lower alkyl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, or wherein R^{a}, R^{b} and R^{c} may be connected by aromatic, aliphatic, heteroaromatic, heteroaliphatic ring structures or substituted embodiments thereof.

The invention also provides combinatorial chemical libraries of purine derivatives. In one preferred embodiment, 6-chloropurine is converted into adenine derivatives by amination of the C6 position of the purine ring; these libraries are termed "N6 libraries" herein. In other preferred embodiments, unsubstituted adenine or 6-chloropurine is derivatized at the N9 position of the purine ring; these libraries are termed "N9 libraries" herein. In still further embodiments, both the C6 and N9 positions are derivatized, with the C6 position being aminated with an amine or substituted amino group; these libraries are termed "N6/N9 libraries" herein.

In the preparation of the N6 or N9 libraries, the starting purine ring structure is reacted in individual "pots" or reaction mixtures with each of a plurality of amines or substituted amines (for N6 libraries) or halides (for N9 libraries). These libraries thus are provided as collections of separate products of the reaction between the starting materials. For N6/N9 libraries, most preferably the N9 position is first derivatized followed by reaction at the C6 position. In these libraries, reaction is typically performed using a single halide (resulting in uniform substitution at the N9 position) and a plurality of amines (preferably 2 to 5 amines, most preferably 3 different amines), thereby providing a mixture of compounds. In addition, regioisomers (including the N1, N3, and N7 isomers) can be produced according to the methods of the invention. Typically, reaction mixtures are also provided lacking the purine starting material, to monitor for reactions between the halides and the different amines.

The invention also provides so-called "rational design" adenine DNA methyltransferase inhibitors, based on an understanding of the putative active site of an adenine DNA methyltransferase enzyme, shown in Figure 1. As schematically depicted in the Figure, the enzyme has a binding site for the adenine residue in a DNA strand, and an S-adenosylmethionine binding site, which provides the donor methyl group as shown. So-called "rational design" inhibitors mimic the configuration of the molecules in the binding site of the enzyme, as shown in Figure 2. These compounds in general comprise an adenosine residue, with or without a 5' phosphate group, covalently linked through a methylene bridge to a homocysteine moiety.
The invention also provides adenine DNA methyltransferase inhibitors that are derivatives of borinic acid, most preferably diphenyl or substituted diphenyl borinic acid, and most preferably diphenyl or substituted diphenyl borinic acid alkylamine esters thereof. In preferred embodiments, the invention provides compounds including di-(p-fluorophenyl)borinic acid 8- hydroxyquiniline ester, di-(p-chlorophenyl)borinic acid 8-hydroxyquiniline ester, diphenylborinic acid 8-hydroxyquiniline ester, di-(p-fluorophenyl)borinic acid ethanolamine ester, and di-(p-chlorophenyl)borinic acid ethanolamine ester.

The invention also provides adenine DNA methyltransferase inhibitors synthesized using solid phase chemistry, most preferably using resins comprising a residue (such as an amine or halide) as provided herein for substitution at the C6 or N9 positions of the purine ring. In preferred embodiments, these resins are provided whereby the substituent is covalently linked to the resin using a covalent bond that can be specifically cleaved to liberate the compound from the resin after solid phase synthesis is complete. Preferably, the substituent is presented on the resin with an activated group, such as an amine or halide, accessible to a purine contacted with the resin. After reaction, the purine is linked to the resin through the substituent, and the reaction product can then be worked up and removed from the resin using methods well known in the art. *See, for example,* Bunin, 1998, THE COMBINATORIAL INDEX, Academic Press.

In certain situations, compounds of the invention may contain one or more asymmetric carbon atoms, so that the compounds can exist in different stereoisomeric forms. These compounds can be, for example, racemates or optically active forms. In these situations, the single enantiomers, *i.e.*, optically active forms, can be obtained by asymmetric synthesis or by resolution of the racemates. Resolution of the racemates can be accomplished, for example, by conventional methods such as crystallization in the presence of a resolving agent, or chromatography, using, for example a chiral HPLC column.

Advantageously, solid phase chemistry employing resins as described above can be useful for determining whether a substituent exhibits chirality or stereospecificity that has a bearing on antibacterial activity. In these embodiments, compounds are prepared for screening using a racemic mixture of optically-active species, such as an amino acid. Upon finding the resulting compound has adenine DNA methyltransferase inhibitory activity, optically-pure preparations of each of the stereoisomers can be used to prepare the corresponding optically-pure isomers of the adenine DNA methyltransferase inhibitory compound, to determine whether there is any difference in biological activity between the isomers. This approach is advantageous over the alternative, separating the racemic mixture into its stereoisomeric components.

Regardless of how a putative adenine DNA methyltransferase is prepared according to the invention, the compound is analyzed for both adenine and cytosine-specific DNA methyltransferase activity. Susceptible bacteria (known to express an adenine DNA methyl transferase) are grown in the presence and absence of the inhibitory compound, and the extent of growth inhibition in the presence of the compound is determined relative to growth in the absence of the compound. The mechanism of action (*i.e.*, inhibition of adenine DNA methyltransferase) is verified for each growth-inhibitory compound by filter-binding radioassay using hemimethylated DNA, tritiated *S*-adenosyl methionine (C³H₃) and a purified adenine DNA methyltransferase according to International Application Publication No. WO98/12206.

Compounds of the invention can exist as tautomers in solution. When structures and names are given for one tautomeric form the other tautomeric form is also included in the invention.

Representative compounds of the present invention include, but are not limited to the compounds disclosed herein and their pharmaceutically acceptable acid and base addition salts. In addition, if the compound of the invention is obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds.

The present invention also encompasses the acylated prodrugs of the compounds of the invention. Those skilled in the are will recognize various synthetic methodologies which may be employed to prepare non-toxic pharmaceutically acceptable addition salts and acylated prodrugs of the inventive compounds.

By "alkyl", "lower alkyl", and "C₁-C₆ alkyl" in the present invention is meant straight or branched chain alkyl groups having 1-6 carbon atoms, such as, methyl, ethyl, propyl, isopropyl, *n*-butyl, *se*c-butyl, *tert*-butyl, pentyl, 2-pentyl, isopentyl, neopentyl, hexyl, 2-hexyl, 3-hexyl, and 3-methylpentyl.

By "alkoxy", "lower alkoxy", and "C₁-C₆ alkoxy" in the present invention is meant straight or branched chain alkoxy groups having 1-6 carbon atoms, such as, for example, methoxy, ethoxy, propoxy; isopropoxy, *n*-butoxy, *sec*-butoxy, *tert*-butoxy, pentoxy, 2-pentyl, isopentoxy, neopentoxy, hexoxy, 2-hexoxy, 3-hexoxy, and 3-methylpentoxy.

By the term "halogen" in the present invention is meant fluorine, bromine, chlorine, and iodine.

By "cycloalkyl", e.g., C₃-C₇ cycloalkyl, in the present invention is meant cycloalkyl groups having 3-7 atoms such as, for example cyclopropyl, cyclobutyl, cyclopentyl, cyclohexyl, and cycloheptyl. In the C₃-C₇ cycloalkyl groups, preferably in the C₅-C₇ cycloalkyl groups, one or two of the carbon atoms forming the ring can optionally be replaced with a hetero atom. such as sulfur, oxygen or nitrogen. Examples of such groups are piperidinyl, piperazinyl, morpholinyl, pyrrolidinyl, imidazolidinyl, oxazolidinyl, azaperhydroepinyl, oxazaperhydroepinyl, oxepanyl, oxazaperhydroinyl, and oxadiazaperhydroinyl. C₃ and C₄ cycloalkyl groups having a member replaced by nitrogen or oxygen include aziridinyl, azetidinyl, oxetanyl, and oxiranyl.

By "aryl" is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which is optionally mono-, di-, or trisubstituted with, e.g., halogen, lower alkyl, lower alkoxy, lower alkylthio, trifluoromethyl, lower acyloxy, aryl, heteroaryl, and hydroxy. Preferred aryl groups include phenyl and naphthyl, each of which is optionally substituted as defined herein.

By "heteroaryl" is meant one or more aromatic ring systems of 5-, 6-, or 7-membered rings containing at least one and up to four heteroatoms selected from nitrogen, oxygen, or sulfur. Such heteroaryl groups include, for example, thienyl, furanyl, thiazolyl, imidazolyl, (is)oxazolyl, pyridyl, pyrimidinyl, (iso)quinolinyl, napthyridinyl, benzimidazolyl, benzoxazolyl. Preferred heteroaryls are thiazolyl, pyrimidinyl, preferrably pyrimidin-2-yl, and pyridyl. Other preferred heteroaryl groups include 1-imidazolyl, 2-thienyl, 1-, or 2- quinolinyl, 1-, or 2- isoquinolinyl, 1-, or 2-tetrahydro isoquinolinyl, 2- or 3- furanyl and 2- tetrahydrofuranyl.

The bacterial growth inhibitory, adenine DNA methyltransfcrase inhibiting compounds of the invention are provided either from combinatorial libraries, solid phase synthesis, "rational" drug design, or conventional synthesis as described herein.

### Construction of Combinatorial Libraries

Combinatorial libraries are prepared according to methods understood by those with skill in the art. For the single substitution libraries (N6 and N9), the individual substituents are used in separate reaction mixtures to produce each of the purine derivatives described herein. In the combination libraries (N6/N9 herein), on the other hand, one position (typically N9) is typically reacted with a particular substituent, and then a mixture of substituents (most preferably 3) used to derivatize the other reaction position (typically C6).

The reactions are performed on a scale adapted to economically producing sufficient product for testing. Preferably, reactions are performed in parallel, for example using a 96-well plate with each well having a sufficiently small volume (100-500µL) to minimize the amount of reagents required. The use of this type of reaction vessel also facilitates parallel handling and analysis, including automated versions of such processes.

### a. N6 Libraries

The following conditions were developed to synthesize analogues of adenine substituted at the N-6 position. In one reaction scheme, 6-chloropurine is reacted at 85°C overnight with a primary or secondary amine in triethylamine in *n*-butanol. Alternatively, these reagents are reacted in potassium carbonate in dimethylformamide at 85°C overnight. This synthesis is described in Reaction Scheme 1. 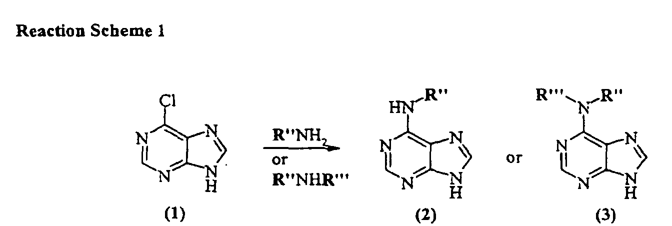 R" and R"' are lower alkyl, hetero atom-substituted lower alkyl, aryl, heteroaryl or substituted aryl or heteroaryl, as exemplified by the compounds set forth below. Any primary or secondary amine can be used in this reaction. Preferred embodiments of primary or secondary amines used in these reactions is as follows:
histamine dihydrochloride
norphenylephrine hydrochloride
1,2-diaminopropane
5-amino-1,3,3-trimethylcyclohexanemethyl-amine
3-isopropoxypropylamine
diphenylborinic acid, cthanolamine ester
2-(2-aminoethylamino)-ethanol
tetrahydrofurfurylamine
5-methyltryptamine hydrochloride
3,3-diphenylpropylamine
1-(3-aminopropyl}-2-pyrrolidinone
2-(2-aminoethyl)-1-methylpyrrolidine
2-(aminomethyl)benzimidazole dihydro-chloridehyrate
2,2,2-trifluoroethylamine hydrochloride
L-camosine
(R)-(-)-1 -ammo-2-propanol
2-(1-cyclohexenyl)ethylamine
4-(trifluoromethyl)benzylamine
2,5-dichloroamylamine hydrochloride
(+/-)-4-amino-3-hydroxybutyric acid
N,N-dimethylethylenediamine
3,3-dimethylbutylamine
1,4-diamino-2-butanone dihydrochloride
aminomethylbenzoic acid
aminohydroxymethylpropane diol
2-(aminoethyl)pyridine
aminobutanol
adamantamine
aminohexanoic acid
N-benyzylethanolamine
ethyl-6-aminobutyrate hydrochloride
ethylenediamine
2-cyclohex-1-enylethylamine

Some of these amines produce different regioisomers, *i.e.*, for some compounds the amine can be added to the C6 position of 6-chloropurine in different orientations, depending on which reactive moiety comprising the amine covalently bonds to C6. However, the occurrence of these regioisomers is not deleterious, since it merely increases the number of candidate compounds in the library.

### b. N9 Libraries

N9 libraries were prepared using the following reaction schemes. it was found that Path A of Reaction Scheme 2 did not yield product with all organic halides (R^{iv}X or R⁴X); alternative Path B was found to form product throughout the range of organic halides tested. In each alternative, the organic halide was reacted with purine (either adenine or 6-chloropurine) at 45°C overnight in potassium carbonate in dimethylformamide. In Path B, however, the N9-derivatized 6-chloropurine was converted to N9-derivatized adenine by reaction of the product of the first reaction with ammonium hydroxide at 85°C overnight. Both reactions are performed sequentially in the same reaction mixture. 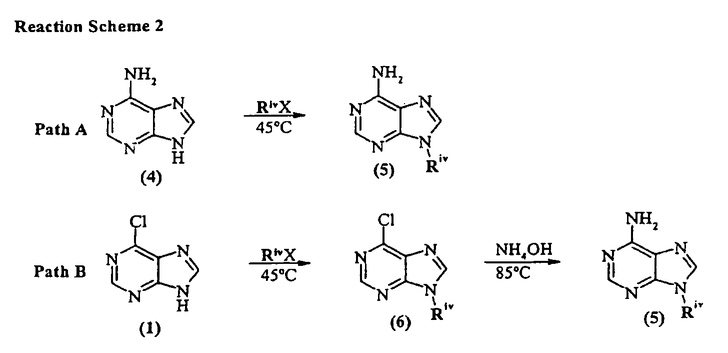 R^{iv} is lower alkyl, hetero atom-substituted lower alkyl, aryl, heteroaryl or substituted aryl or heteroaryl, as exemplified by the compounds set forth below.
The products of Path B were analyzed by HPLC and found to be a mixture of N-9 and N-7 substituted adenine analogues; there may also be N-1 and N-3 substituted analogues in certain reaction mixtures. As discussed above, the advantage of these side products is that their existence simply increases the number of candidate molecules in the library.

Any organic halide can be used in this reaction. Preferred embodiments of organic halides used in these reactions is as follows:
methyl 4-iodobutyrate
1-bromo-3-phenylpropane
cinnamyl bromide
2-chloroethylphosphonic acid
ethyl 2-(2-chloroacetamido)-4-thiazote-acetate
4-(2-chloroethyl)morpholine hydrochloride
(2-bromoethyl)trimethylammonium bromide
4-chlorophenyl 2-bromoethyl ether
N-(3-bromopropyl)pthalimide
2-chloroethyl isocyanate
2-chloro-N, N-dimethylacetoacetamide
3-chloro-2-hydroxypropyl methacrylate
2-bromo-2'-hydrozy-5'-nitroacetanilide
3-(2-bromoethyl)indole
5-chloro-2-pentanone ethylene ketal
2-chloroethyl ethyl sulfide
3-chloro-N-hydroxy-2,2-dimethyl-propionamide
L-1-p-Tosylamino-2-Phenylethyl chloromethyl ketone
2-(2-bromoethyl)-1,3-dioxolane
ethyl 2-(bromomethyl)acrylate
2-(2-(2-chlorethyoxy)ethoxy)ethanol
(3-chloropropyl)trimethoxysilane
chloramphenicol
4-(chloromethyl)benzoic acid
bromoethylamine hydrochloride
epibromohydrin
iodopentane
benzyl bromide

### c. N6/N9 Combination Libraries

Having developed the reaction schemes shown above for N6 and N9 libraries, 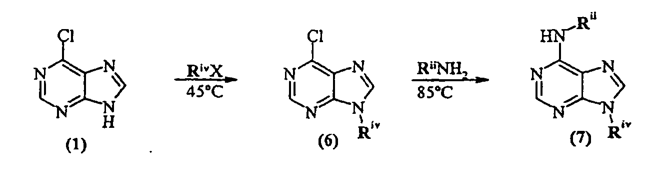 combination libraries having substituents at both the C6 amino and N9 positions were prepared using a modification of Path B of Reaction Scheme 2.

6-Chloropurine was reacted with an organic halide as described above at 45°C overnight in a solution of potassium carbonate in dimethylformamide. Thereafter, a primary or secondary amine is added to the reaction mixture (although the scheme depicts a primary amine, primary or secondary amines are useful in Reaction Scheme 3) and compound (7) prepared by reaction at 85°C overnight. In this modification, the primary or secondary amine is substituted in the reaction for ammonium hydroxide show in Path B of Reaction Scheme 2.

Any organic halide can be used in the first step of this reaction. Preferred embodiments of organic halides used in these reactions is as follows:
methyl 4-iodobutyrate
1-bromo-3-phenylpropane
cinnamyl bromide
2-chloroethylphosphonic acid
ethyl 2-(2-chloroacctamido)-4-thiazole-acetate
4-(2-chloroethyl)morpholine hydrochloride
(2-bromoethyl)trimethylammonium bromide
4-chlorophenyl 2-bromoethyl ether
N-(3 -bromopropyl)pthalimide
2-chloroethyl isocyanate
2-chloro-N, N-dimethylacetoacetamide
3-chloro-2-hydroxypropyl methacrylate
2-bromo-2'-hydroxy-5'-nitroacetanilide
3-(2-bromoethyl)indole
5-chloro-2-pentanone ethylene ketal
2-chloroethyl ethyl sulfide
3-chloro-N-hydroxy-2,2-dimethyl-propionamide
L-1-p-Tosylamino-2-Phcnylethyl chloromethyl ketone
2-(2-bromoethyl)-1,3 -dioxolane
ethyl 2-(bromomethyl)acrylate
2-(2-(2-chlorethyoxy)ethoxy)ethanol
(3-chloropropyl)trimethoxysilane
chloramphenicol
4-(chloromethyl)benzoic acid
bromoethylamine hydrochloride
epibromohydrin
iodopentane
benzyl bromide

Any primary or secondary amine can be used in the second step of this reaction. Preferred embodiments of primary or secondary amines used in these reactions is as follows:
histamine dihydrochloride
norphenylephrine hydrochloride
1,2-diaminopropane
5 -amino- 1,3,3 -trimethylcyclohexanemethyl-amine
3-isopropoxypropylamine
diphenylborinic acid, ethanolamine ester
2-(2-aminoethylamino)-ethanol
tetrahydrofurfurylamine
5-methyltryptamine hydrochloride
3,3-diphenylpropylamine
1-(3-aminopropyl)-2-pyrrolidinone
2-(2-arninoethyl)-1-methylpyrrolidine
2-(aminomethyl)benzimidazole dihydro-chloridehyrate
2,2,2-trifluoroethylamine hydrochloride
L-carnosine
(R)-(-)-1-amino-2-propanol
2-(1 -cyclohexenyl)ethylamine
4-(trifluoromethyl)benzylamine
2,5-dichloroamylaminc hydrochloride
(+/-)-4-amino-3-hydroxybutyric acid
N,N-dimethylethylenediamine
3,3-dimethylbutylamine
1,4-diamino-2-butanone dihydrochloride
aminomethylbenzoic acid
aminohydroxymethylpropane diol
2-(aminoethyl)pyridine
aminobutanol
adamantamine
aminohexanoic acid
N-benyzylethanolamine
ethyl-6-aminobutyrate hydrochloride
ethylenediamine
2-cyclohex-1-enylethylamine

This chemistry can be repeated with any halide in combination with any amine to give any adenine analogues of this type.

As with Reaction Schemes 1 and 2, different regioisomers are produced with certain N6 amines, and substituted purines are produced at the N1, N3 and N7 positions as well as at the N9 position by reaction with organic halides.

To obtain a complete combinatorial library in a reasonable time the chemistry was performed in a 96 well plate using 80 wells at a time (Columns 1-10, Rows A-H). A single halide was added to the reaction mixture in each row and the first portion of the reaction shown in Reaction Scheme 3 performed overnight as describe above. Sets of three different amines were then added to each well in each of columns 1-7; wells in columns 8, 9 and 10 are prepared containing only one amine, particularly for those species that would have a tendency to react with any additional amines in the reaction mixture. The second portion of the reaction performed overnight. As a result, most of the wells contained a mixture of compounds, each well comprising a set of purine derivatives with a particular substituent at the N9 position and one of three different substituted amino groups at C6. It is recognized that certain of the reaction mixtures will be deficient in one, two or all three of the C6 substituents, depending on the reactivity of each amino group with N9-derivatized 6-chloropurine. In addition, the reaction mixtures contain different regioisomers of these products. Finally, reaction between combinations of the amines reacting directly with the halides without reacting with 6-chloropurine is possible. These product compounds in each reaction mixture were tested as mixtures for antibacterial, and specifically adenine DNA methyltransferase inhibitory activity. Mixtures showing positive results were separated to identify the compound responsible for the result

### Screening of Combinatorial Libraries

Reaction products from each of the libraries prepared as described above were screened
using both *in vivo* and *in vitro* screening methods.

*In vivo* screening methods involved assays for growth inhibition of bacterial cells expressing an adenine DNA methyltransferase essential for cell growth. Advantageously, these screening methods utilize more than one species of bacteria, to identify lead candidates having the broadest spectrum of antibiotic activity. In certain embodiments, the putative inhibitors are first screened against samples of gram positive and gram-negative bacteria; *Caulobacter cresentus* and *Bacillus subtilis* are advantageous examples. Additionally advantageous bacterial species for detecting *in vivo* adenine DNA methyltransferase activity include *Helicobacter pylori, Agrobacter tumefaciens, Brucella abortus* and *Bacillus anthracis*

In these assays, bacterial cultures such as *Caulobacter* were grown in an appropriate bacterial culture media such as peptone yeast extract (PYE) media (DIFCO) overnight to saturation. Aliquots of this culture were diluted to a concentration having an optical density at 600nm (OD₆₀₀) of about 0.05. The assay is conveniently performed in 96 well microtitre plates, particularly using libraries prepared in such plates. Using these microtitre plates, an equal amount (100-500µL) of the diluted bacterial culture was placed in 88 of the 96 wells of the microtitre plate; the remaining 8 wells were used as negative (no bacteria) controls. Eight of the wells were used as positive (no added test compound) controls. For library screening, bacterial aliquots of 146µl. can be used per well with the addition of 4µL of combinatorial library sample.

A different mixture of library compounds was added to each of the remaining 80 wells per plate, and the cells grown for 24h at 37°C. Bacterial cell growth was monitored at intervals using a microplate reader to monitor cell growth; cell growth can be monitored by measuring the OD₆₃₀. Wells containing cells growing more slowly than control wells were used to identify corresponding combinatorial library reaction mixtures, which were then synthesized and tested individually to determine the identity of the inhibitory compound.

Using these methods, candidate compounds that inhibited bacterial cell growth at an estimated concentration of <100µM were identified. Candidate compounds identified from these libraries include 6-N-(diphenylborinic ester)-ethyl-adenine, 6-N-(diphenylborinic ester)-ethyl-9-(2-(4-morpholinyl)-ethyl)-adenine, 6-N-(diphenylborinic ester)-ethyl-9-(3-(N-phthaloyl)-aminopropyl)-adenine, 6-N-(diphenylborinic ester)-ethyl-9-(2-(2-(2-hydroxyethoxy)ethoxy)ethyl)-adenine, and 6-N-(diphenylborinic ester)-ethyl-9-(ethyl-2-acrylate)-methyl-adenine.

*In vitro* assays were performed directly on reaction mixtures from the combinatorial libraries of the invention, or on candidate compounds identified from the in vivo screening assays described above. These assays are of two types, using purified CcrM methyltransferases from *Caulobacter cresentus* or *Brucella abortus,* or using commercially-available preparations of bacterial *dam* methylases and *dcm* methylases. In these assays, a synthetic hemimethylated 45/50 DNA substrate (as disclosed in Berdis *et al.,* 1998, *Proc. Natl. Acad. Sci. USA* 95: 2874-2879) was incubated with the combinatorial library sample containing a putative inhibitor and the methyltransferase at 30°C, and the methyltransferase reaction initiated by the addition of ³H-labeled *S*-adenosylmethionine (wherein the radioisotope label comprises the transferred methyl group). Inhibition is detected by comparing the amount of radiolabel incorporated in controls where the reaction was performed in the presence and absence of combinatorial library samples; inhibitors cause a reduction in the amount of methylated, ³H-labeled DNA collected on a DE81 filter and radioactivity quantified by liquid scintillation.

Using this assay, four additional compounds from the N6 library were detected that completely inhibited *in vitro* DNA methylation at an estimated concentration of about 500µM. These compounds (having the structures shown below) were ribose forms of adenosine having either diphenylborinic acid ethanolamine ester or 3,3-diphenylisopropyl groups covalently linked to the C6 amino group of the adenine ring:

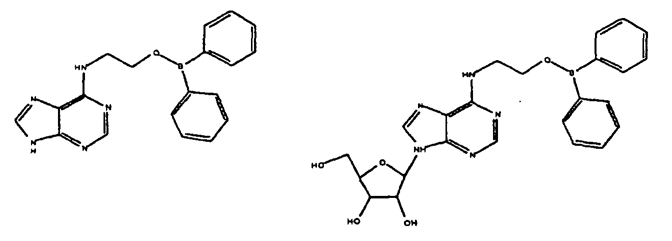

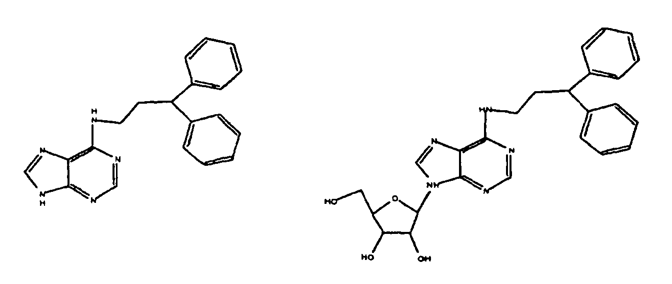

One reaction mixture from the synthesis of the structures shown above from the N-6 adenine library was further analyzed and found to inhibit cell growth at < 50µM. An approximate Kᵢ of 1 µM was measured (assuming the theoretical maximum concentration in the well) for this compound, having the structure:

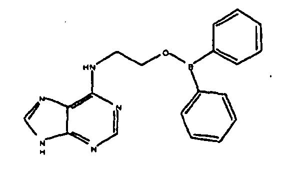

Selected reaction mixtures from the N-9 adenine library were tested for inhibition of adenine DNA methyltransferase activity using this assay. Compounds obtained in the synthesis having N9 substituted with 3-ethylindole or 2-ethyl-1,3-dioxolane (having the structures shown below) were found to be inhibitors of adenine DNA methyltransferase activity at 500 µM:

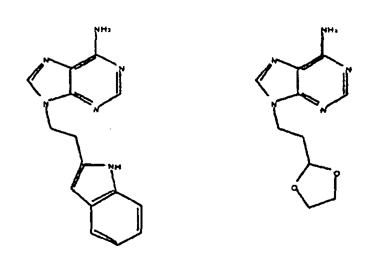

*In vitro* assays using *dcm* methyltransferases were performed essentially as described using commercially-available methyltransferase from *Hemophilus hemolyticus* (New England Biolabs, Beverly, MA) and pUC18 DNA as substrate; this assay can also be performed with other commercially-available *dcm* methyltransferases, *for example,* from *Arthtobacter luteus, Bacillus amyloliquifaciens* H, *Hemophilus aegyptius, Hemophilus parainfluenza,* or *Moraxella* species. In these assays, adenine specificity is demonstrated by detecting little or no inhibition of the *dcm* methyltransferase, so that the amount of methylated, ³H-labeled DNA collected on a DE81 filter and radioactivity quantified by liquid scintillation is the same in the presence or absence of the combinatorial library mixtures or putative adenine-specific inhibitor.

Alternatively, assays using *dam* methylases, for example from *Escherichia coli,* are performed wherein inhibition is demonstrated by a reduction in the amount of methylated, ³H-labeled DNA was collected on a DE81 filter and radioactivity quantified by liquid scintillaton.

### Synthesis of "Rational Design" Adenine DNA Methylase Inhibitors

### a. Active site analogues

"Rational design" of adenine DNA methyltransferases depends on the assumption that the active site of the enzyme contains specific binding sites for the adenine moiety to be modified as well as the *S*-adenosyl methionine methyl donor, as shown in Figure 2. Compounds that fit within the active site of the methyltransferasc are preferred , and molecules that mimic the putative "transition state" where the methyl transfer activity of the enzyme occurs are particularly preferred. Four such transition state analogues were prepared and assayed *in vitro* for adenine DNA methyltransferase activity.

The rational design compounds have the structure: 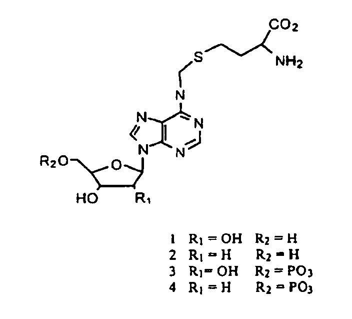 and were synthesized using Reaction Scheme 6, shown below.

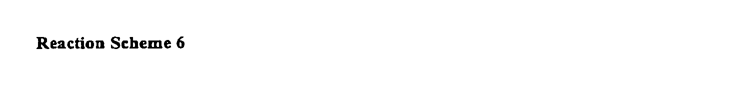

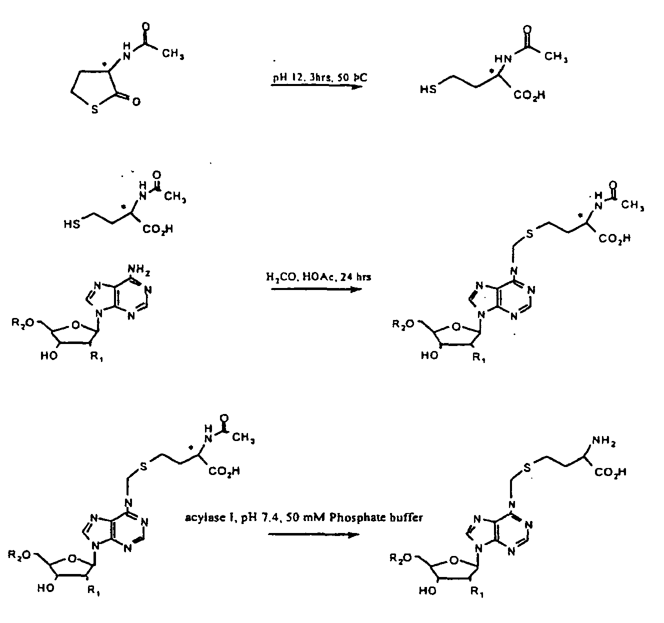

These compounds were assayed as described above for adenine DNA methyltransferase activity. These compounds were found to inhibit CcrM with the Kᵢ's indicated in Table 1. The Kᵢ's for compounds 2 and 4 were calculated from a Dixon plot of the data measured at concentration of inhibitors from 0 - 150 µM for 3 and 0 - 80 µM for compound 4. Kᵢ's for 1 and 2 are estimated from IC₅₀' s.

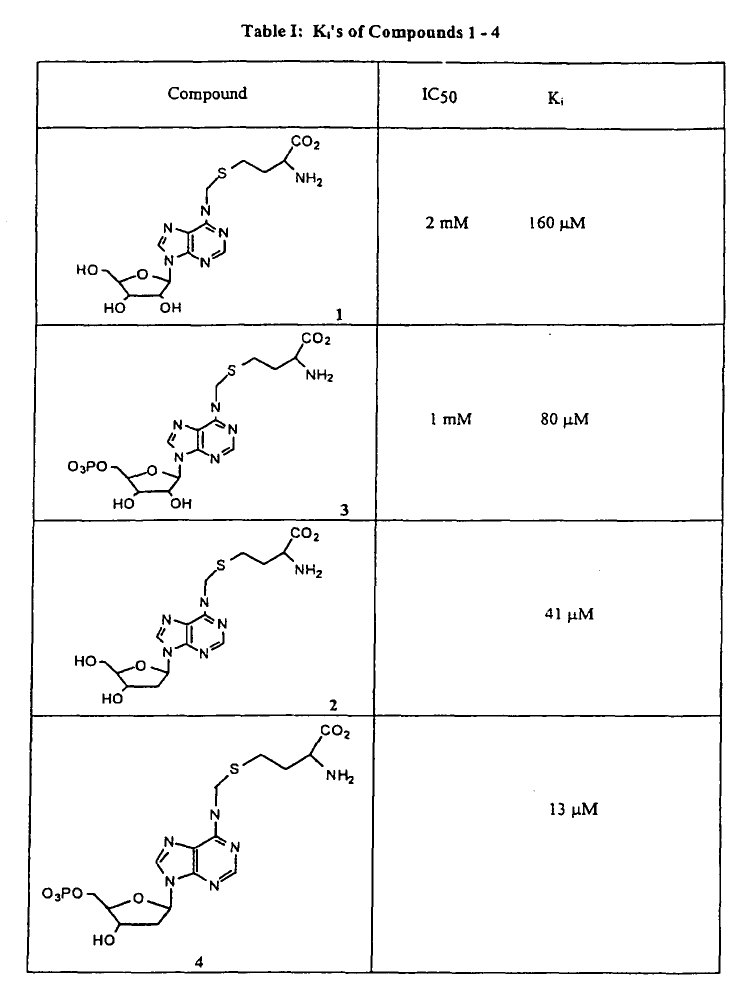

### Solid Phase Synthesis of Adenine DNA Methyltransferase Inhibitors

Adenine DNA methyltransferase inhibitors of the invention are also advantageously synthesized using solid phase synthetic methods well known in the art. *See* Bunin, *ibid.*

In preferred embodiments, solid phase synthesis complements combinatorial library synthesis as described herein by allowing access to a larger number of library compounds for screening. This synthetic method has the additional advantages of being easier to handle and easier to purify, since they are attached to the resin by chemically-labile groups that can be specifically cleaved.

For example, compound (**8**) was identified from the N6 library having relatively low (mM range) inhibitory activity:

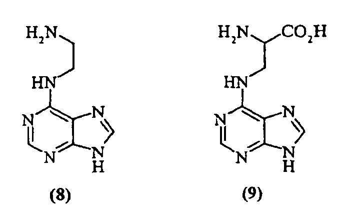

Using these results, second generation libraries were developed using solid phase chemistry to modify inhibitor (**8**) and its related analogue (**9**). For example, compound (**8**) can be derivatized from the terminal amine and from the N-9 position to produce inhibitors that are designed to interact with the *S*-adenosyl methionine (SAM) and DNA binding sites respectively. Such derivatives can be prepared to target either portion of the methyltransferase active site: modifications of the N9 position are specific to the adenine binding site, while modifications of the C6 amine is specific for the SAM site.

Solid phase chemistry was performed using reaction schemes illustrated by Reaction Scheme 4:

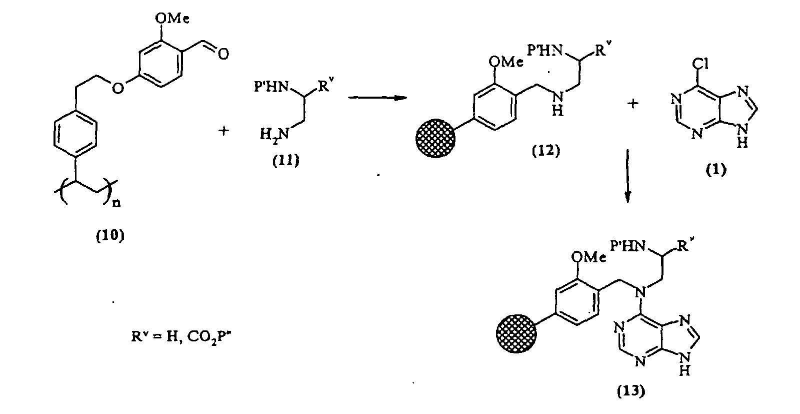

### Reaction Scheme 4

These experiments used a commercially-available methoxyphenyl formyl resin to perform a reductive amination with a protected diamine (**11**), where R^{v} is hydrogen or CO₂P" (where P' and P" are protecting groups). This reaction yields the secondary amine (**12**). Addition of 6-chloropurine results in resin-bound adenine adduct (**13**), permitting the remaining functional groups to be derivatized on the resin. The adducts can be removed from the resin according to art-recognized methods, such as treatment with trifluoroacetic acid, either in concentrated form or as a 5% solution in dichloromethane.

Reaction scheme 4 illustrates an embodiment where the amino substituent contains a chiral center (*i.e.*, it exists as a pair of stereoisomers). However, only one of these stereoisomers may have biological activity. In order to avoid having to separate diastereomeric forms of the compounds of the invention, the following Reaction Scheme 5 can be used:

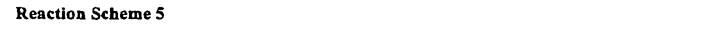

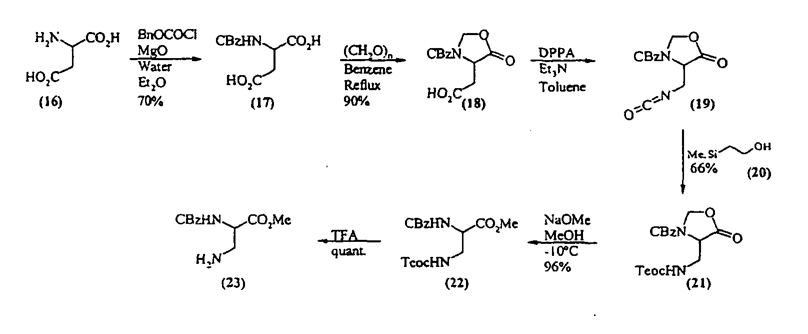

This synthesis can be used to detect adenine DNA methyltransferase inhibitory activity in compounds comprising a racemic mixture of a chiral center (as occurs in aspartic acid (**16**)); this synthesis can be repeated with commercially pure D- or L-aspartic acid to obtain optically-pure embodiments in the event that one stereoisomer has significantly more activity that the other. As shown in Reaction Scheme 5, D,L-Aspartic acid (**16**) was treated with benzyl chloroformate to yield the N-carboxybenzyl protected aspartic acid (**17**). The α-carboxylic acid was then protected as an oxazolidinone (**18**) using paraformaldehyde. A Curtius rearrangement was performed on the remaining β-carboxylic acid using diphenylphosphoryl azide to give the isocyanate (**19**). These reactions comprise art-recognized synthetic methods; from this point forward the chemistry is novel. The isocyanate (**19**) was trapped using trimethylsilyl ethanol (**20**) to give the Teoc (trimethylsilylethoxycarbonyl) protected amine (**21**).The oxazolidinone was ring opened using sodium methoxide to give the methyl ester (**22**). The Teoc protecting group was then removed using trifluoroacetic acid to yield the mono protected diamine (**23**).

Compounds (**23**) and (**11**, where **R**^{**v**} = **H, P'** = **CBz**) have been used to synthesize the resin bound adenine analogues (**13**), having R^{v} = CO₂Me, P' = CBz, and (**13**), R^{v} = H, P' = CBz, respectively.

### Uses of the Compounds of the Invention

The invention also provides embodiments of the compounds disclosed herein as pharmaceutical compositions. The pharmaceutical compositions of the present invention can be manufactured in a manner that is itself known, *e.g*., by means of a conventional mixing, dissolving, granulating, dragee-making, levigating, emulsifying, encapsulating, entrapping or lyophilizing processes.

Pharmaceutical compositions for use in accordance with the present invention thus can be formulated in conventional manner using one or more physiologically acceptable carriers comprising excipients and auxiliaries that facilitate processing of the active compounds into preparations that can be used pharmaceutically. Proper formulation is dependent upon the route of administration chosen.

Non-toxic pharmaceutical salts include salts of acids such as hydrochloric, phosphoric, hydrobromic, sulfuric, sulfmic, formic, toluenesulfonic, methanesulfonic, nitic, benzoic, citric, tartaric, maleic, hydroiodic, alkanoic such as acetic, HOOC-(CH₂)ₙ-CH₃ where n is 0-4, and the like. Non-toxic pharmaceutical base addition salts include salts of bases such as sodium, potassium, calcium, ammonium, and the like. Those skilled in the art will recognize a wide variety of non-toxic pharmaceutically acceptable addition salts.

For injection, the compounds of the invention can be formulated in appropriate aqueous solutions, such as physiologically compatible buffers such as Hanks's solution, Ringer's solution, or physiological saline buffer. For transmucosal and transcutaneous administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees; capsules, liquids, gels, syrups, slurries, suspensions and the like, for oral ingestion by a patient to be treated. Pharmaceutical preparations for oral use can be obtained with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents can be added, such as the cross-linked polyvinyl pyrrolidone, agar, or alginic acid or a salt thereof such as sodium alginate.

Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration. For buccal administration, the compositions can take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of *e.g.*, gelatin for use in an inhaler or insufflator can be formulated containing a powder mix of the compound and a suitable powder base such as lactose or starch.

The compounds can be formulated for parenteral administration by injection, *e.g.*, by bolus injection or continuous infusion. Formulations for injection can be presented in unit dosage form, *eg.*, in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, *e.g.*, sterile pyrogen-free water, before use. The compounds can also be formulated in rectal compositions such as suppositories or retention enemas, *e.g.*, containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system can be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system can be varied considerably without destroying its solubility and toxicity characteristics. Furthermore, the identity of the co-solvent components can be varied: for example, other low-toxicity nonpolar surfactants can be used instead of polysorbate 80; the fraction size of polyethylene glycol can be varied; other biocompatible polymers can replace polyethylene glycol, *e.g.* polyvinyl pyrrolidone; and other sugars or polysaccharides can substitute for dextrose.

Alternatively, other delivery systems for hydrophobic pharmaceutical compounds can be employed. Liposomes and emulsions are well known examples of delivery vehicles or carriers for hydrophobic drugs. Certain organic solvents such as dimethylsulfoxide also can be employed, although usually at the cost of greater toxicity. Additionally, the compounds can be delivered using a sustained-release system, such as semipermeable matrices of solid hydrophobic polymers containing the therapeutic agent. Various sustained-release materials have been established and are well known by those skilled in the art. Sustained-release capsules can, depending on their chemical nature, release the compounds for a few weeks up to over 100 days. Depending on the chemical nature and the biological stability of the therapeutic reagent, additional strategies for protein and nucleic acid stabilization can be employed.

The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

The compounds of the invention can be provided as salts with pharmaceutically compatible counterions. Pharmaceutically compatible salts can be formed with many acids, including but not limited to hydrochloric, sulfuric, acetic, lactic, tartaric, malic, succinic, phosphoric, hydrobromic, sulfinic; formic, toluenesulfonic, methanesulfonic, nitic, benzoic, citric, tartaric, maleic, hydroiodic, alkanoic such as acetic, HOOC-(CH₂)ₙ-CH₃ where n is 0-4, and the like. Salts tend to be more soluble in aqueous or other protonic solvents that are the corresponding free base forms. Non-toxic pharmaceutical base addition salts include salts of bases such as sodium, potassium, calcium, ammonium, and the like. Those skilled in the art will recognize a wide variety of non-toxic pharmaceutically acceptable addition salts.

Pharmaceutical compositions of the compounds of the present invention can be formulated and administered through a variety of means, including systemic, localized, or topical administration. Techniques for formulation and administration can be found in "Remington's Pharmaceutical Sciences," Mack Publishing Co., Easton, PA. The mode of administration can be selected to maximize delivery to a desired target site in the body. Suitable routes of administration can, for example, include oral, rectal, transmucosal, transcutaneous, or intestinal administration; parenteral delivery, including intramuscular, subcutaneous, intramedullary injections, as well as intrathecal, direct intraventricular, intravenous, intraperitoneal, intranasal, or intraocular injections.

Alternatively, one can administer the compound in a local rather than systemic manner, for example, via injection of the compound directly into a specific tissue, often in a depot or sustained release formulation.

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used in the method of the invention, the therapeutically effective dose can be estimated initially from cell culture assays, as disclosed herein. For example, a dose can be formulated in animal models to achieve a circulating concentration range that includes the EC50 (effective dose for 50% increase) as determined in cell culture, *i.e.*, the concentration of the test compound which achieves a half-maximal inhibition of bacterial cell growth. Such information can be used to more accurately determine useful doses in humans.

It will be understood, however, that the specific dose level for any particular patient will depend upon a variety of factors including the activity of the specific compound employed, the age, body weight, general health, sex, diet, time of administration, route of administration, and rate of excretion, drug combination, the seventy of the particular disease undergoing therapy and the judgment of the prescribing physician.

For administration to non-human animals, the drug or a pharmaceutical composition containing the drug may also be added to the animal feed or drinking water. It will be convenient to formulate animal feed and drinking water products with a predetermined dose of the drug so that the animal takes in an appropriate quantity of the drug along with its diet. It will also be convenient to add a premix containing the drug to the feed or drinking water approximately immediately prior to consumption by the animal.

Preferred compounds of the invention will have certain pharmacological properties. Such properties include, but are not limited to oral bioavailability, low toxicity, low serum protein binding and desirable *in vitro* and *in vivo* half lives, Assays may be used to predict these desirable pharmacological properties. Assays used to predict bioavailability include transport across human intestinal cell monolayers, including Caco-2 cell monolayers. Serum protein binding may be predicted from albumin binding assays. Such assays are described in a review by Oravcová *et al.* (1996, *J. Chromat. B* 677: 1-27). Compound half life is inversely proportional to the frequency of dosage of a compound. *In vitro* half-lives of compounds may be predicted from assays of microsomal half-life as described by Kuhnz and Gieschen (Drug Metabolism and Disposition, (1998) volume 26, pages 1120-1127).

Toxicity and therapeutic efficacy of such compounds can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio between LD50 and ED50. Compounds that exhibit high therapeutic indices are preferred. The data obtained from these cell culture assays and animal studies can be used in formulating a range of dosage for use in humans. The dosage of such compounds lies preferably within a range of circulating concentrations that include the ED50 with little or no toxicity. The dosage can vary within this range depending upon the dosage form employed and the route of administration utilized. The exact formulation, route of administration and dosage can be chosen by the individual physician in view of the patient's condition. (*See, e.g.* Fingl *et al.,* 1975, in "The Pharmacological Basis of Therapeutics", Ch.1, p.1).

Dosage amount and interval can be adjusted individually to provide plasma levels of the active moiety that are sufficient to maintain bacterial cell growth inhibitory effects. Usual patient dosages for systemic administration range from 100 - 2000 mg/day. Stated in terms of patient body surface areas, usual dosages range from 50 - 910 mg/m²/day. Usual average plasma levels should be maintained within 0.1-1000 µM. In cases of local administration or selective uptake, the effective local concentration of the compound cannot be related to plasma concentration.

The compounds of the invention are modulators of cellular processes in bacteria that infect plants, animals and humans. The pharmaceutical compositions of the adenine DNA methyltransferase inhibitory compounds of the invention are useful as antibiotics for the treatment of diseases of both animals and humans, including but not limited to actinomycosis, anthrax, bacterial dysentery, botulism, brucellosis, cellulitis, cholera, conjunctivitis, cystitis, diphtheria, bacterial endocarditis, epiglottitis, gastroenteritis, glanders, gonorrhea, Legionnaire's disease, leptospirosis, bacterial meningitis, plague, bacterial pneumonia, puerperal sepsis, rheumatic fever, Rocky Mountain spotted fever, scarlet fever, streptococcal pharyngitis, syphilis, tetanus, tularemia, typhoid fever, typhus, and pertussis.

The disclosures in this application of all articles and references, including patents, are incorporated herein by reference.

The following Examples are provided for the purposes of illustration and are not intended to limit the scope of the present invention. The present invention is not to be limited in scope by the exemplified embodiments, which are intended as illustrations of individual aspects of the invention. Indeed, various modifications of the invention in addition to those shown and described herein will become apparent to those skilled in the art from the foregoing description and accompanying drawings. Such modifications are intended to fall within the scope of the appended claims.

### EXAMPLE 1

### Preparation of Solution Phase Chemistry Libraries

Solution phase combinatorial libraries as described above were prepared in a 96-well microtitre plate as follows.

To each well in columns 1-7 was added K₂CO₃ (7-10 mg) followed by DMF (140 µL) and the 6-chloropurine (30 µL of 0.5M solution in DMF). To each row was added a halide (15 µL of a 1M solution in DMF) selected from the list of halides disclosed herein.

To each well in columns 8-10 was added K₂CO₃ (3-5 mg) followed by DMF (180 µL) and the 6-chloropurine (10 µL of 0.5M solution in DMF). To each row was added a halide (5 µL of a 1M solution in DMF) selected from the list of halides disclosed herein.

The microtitre plate was heated to 45°C and reacted overnight. Reactions were cooled to room temperature and the second synthetic reaction performed as follows.

To each well in columns 1-7 was added 3 different amines(5 µL each of a 1M solution in DMF ) selected from the list of amines dislosed herein.

To each well in columns 8-10 was added one amine (5 µL each of a 1M solution in DMF) selected from the list of amines dislosed herein.

The microtitre plate was heated to 85°C and reacted overnight. Reactions were cooled to room temperature, each well was collected separately and the final volume of the solution was adjusted to 300 µL (replacing solvent lost to evaporation).

The compounds produced in these reactions were then tested using the in vivo bacterial growth assays disclosed herein.

### EXAMPLE 2

### Preparation of Adenine DNA Methyltransferase Inhibitors

The combinatorial libraries of the invention were screened for adenine DNA methyltransferase inhibitory activity as described above. A compound displaying methyltransferase inhibiting activity and having the C6 amino group covalently linked to diphenylborinic acid ethanolamine ester was used as the base compound for preparing related analogues according to the following reaction scheme:

Specifically, 6-chloropurine (**1**) was dissolved in dry DMF (about 0.3mmol/mL)

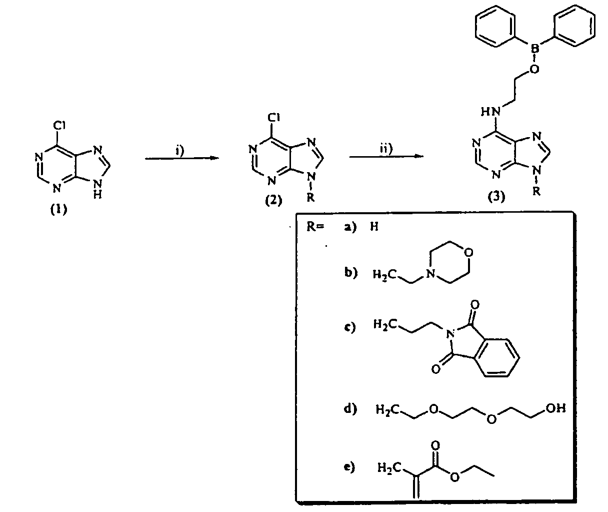

Conditions i) R-X, DMF, K₂CO_{3,}45-95 °C; ii) Ph₂BOCH₂CH₂NH₂, DMF, K₂CO_{3,} 90-95 °C under argon at room temperature. Potassium carbonate (K₂CO₃; 2-3 equivalents) was added, followed by one equivalent of the alkyl halide (R-X). The reaction was heated to either 45°C or 95°C (if the halide does not react at the lower temperature) and stirred for 18 hours. After this time, thin layer chromatography was performed (using 2%- 5% methanol in dichloromethane as solvent) and showed little or no starting material remaining in the reaction mixture. The reaction was cooled to room temperature and the solid removed by filtration and washed with dry dimethylformamide (DMF). The filtrate was obtained and any remaining DMF was removed *in vacuo* to give the crude product as an oil. The product was purified by column chromatography on silica gel (using 2%-5% methanol in dichloromethane as solvent). The N-9 regioisomer was eluted as a pure fraction before a combination of the N-9 and N-7 regioisomers that eluted as a mixture. The fractions containing the pure N-9 isomer were combined and the solvent was removed *in vacuo* to give the intermediate product **2b - 2e** as a solid or an oil that solidified on standing.

The final compounds were prepared as follows. 6-chloropurine (1) or 9-alkyl-6-chloropurine (**2b - 2e**) was dissolved in dry DMF (0.03-0.5 mmol/mL) under argon at room temperature. Potassium carbonate (K₂CO₃; 1.5 - 2 equivalents) was added followed by one equivalent of diphenylborinic acid ethanolamine ester. The reaction was heated to 90-95°C and stirred for 18 hours. The mixture was allowed to cool to room temperature and the solid was removed by filtration as described above. The structure of these compounds was confirmed using ¹H-NMR, ¹³C-NMR, and two-dimensional NMR spectroscopic methods such as HMQC and HMBC.

Alternatively, 6-chloropurine (**1**) or N-9 alkyl-6-chloropurine (**2b - 2e**) was dissolved in 1-butanol (0.1mmol/mL) under argon at room temperature. Three equivalents of diisopropylethylamine were added, followed by the addition of 1.2 equivalents of alkyl halide. This reaction mixture was heated to 110°C and stirred for 18 hours. The reaction was then cooled to room temperature and the solvent was removed in *vacuo.* The residue was purified by column chromatography on silica gel (using a solution of 2% to 5% methanol in dichloromethane in solvent). The product fractions were collected and the solvent was removed *in vacuo* to leave a white solid.

It is known in the art that the structure of the staring material, diphenylborinic acid ethanolamine ester, is cyclic and the boron is tetrahedral. Thus, cyclic and linear analogues of the adenine DNA methyltransferase inhibiting compounds of the invention may be advantageous for developing additional inhibitory compounds.

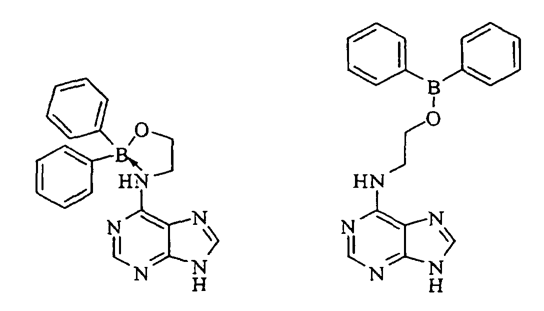

For *in vivo* assays, the unpurified preparation, which contained residual DMF or dimethylsulfoxide (DMSO), was diluted to 16.7mM and used directly as a crude mixture.

*In vivo* assays of bacterial cell growth inhibition were performed essentially as described above using a variety of bacterial species. The compounds (**3a**) through (**3e**) showed the following results in these assays:

### Caulobacter cresentus

Compound **3a** - IC₅₀ <25µM
Compound **3b** - IC₅₀ <25µM
Compound **3c** - IC₅₀ <25µM
Compound **3d** - IC₅₀ <25µM
Compound **3e**-IC₅₀ <25µM

### Brucella abortus

Compound **3a** - Almost complete cell death after 12 hours - 100µM
Compound **3b** - Complete cell death after 12 hours - 100µM
Compound **3c** - Cell growth inhibited from start - 100µM
Compound **3d** - Cell growth inhibited from start - 100µM
Compound **3e** - Cell growth inhibited from start - 100µM

### Helicobacter pylori

Compound **3a** - IC₅₀ <25µM
Compound **3b** - IC₅₀ <25µM
Compound **3c** - IC₅₀ between 25-100µM
Compound **3d** - IC₅₀ between 25-100µM
Compound **3e** - IC₅₀ = 25µM

### Agrobacterium tumefaciens

Compound **3a** - IC₅₀ > 100µM
Compound **3b** - IC₅₀ = 25µM
Compound **3c** - IC₅₀ = 25 µM
Compound **3d** - IC₅₀ <<25µM
Compound **3e** - IC₅₀ << 25µM

### Bacillus subtilis

Compound **3a** - IC₅₀ between 10-50µM
Compound **3b** - IC₅₀ between 1-10µM
Compound **3c** - IC₅₀ between 1-10µM
Compound **3d** - IC₅₀ between 10-50µM
Compound **3e** - not tested

In addition, the following results were obtained using *in vitro* adenine DNA methyltransferase inhibition assays:

### CcrM

Compound **3a** - Complete inhibition at 100µM
Compound **3b** - Complete inhibition at 100µM
Compound **3c** - Complete inhibition at 100µM
Compound **3d** - Complete inhibition at 100µM
Compound **3e** - Complete inhibition at 100µM

### dam methylase (E. coli)

Compound **3a** - Complete inhibition at 100µM
Compound **3b** - Complete inhibition at 100µM
Compound **3c** -Complete inhibition at 100µM
Compound **3d** - Complete inhibition at 100µM
Compound **3e** - Complete inhibition at 100µM

### dcm methyltransferase (HhaI)

Compound **3a** - No inhibition at all at 500µM
Compound **3b** - No inhibition at all at 500µM
Compound **3c** - No inhibition at all at 500µM
Compound **3d** - No inhibition at all at 500µM
Compound **3e** - No inhibition at all at 500µM

These results demonstrate that compounds (**3a**) through (**3e**) are adenine-specific DNA methyltransferases with no detectable *dcm* crossreactivity.

### EXAMPLE 3

### Preparation of Adenine DNA Methyltransferase Inhibitors

Additional adenine DNA methyltransferase inhibitors were developed by optimization of leads found during screening of the combinatorial libraries described above.

### 1. 6-(2-diphenylmethylcyclopentylamino)purine (Compound 73)

6-chloropurine was combined with S-(-)-2-(diphenylmethyl)-pyrrolidine in n-butanol with two equivalents of diisopropylethylamine (N(iPr)₂Et). The reaction was heated to 105□C and allowed to react for 24 h. Solvent was removed from the reaction mixture *in vacuo* and the crude product purified by silica gel chromatography.

### 2. 6-(2-diphenylhydroxymethylcyclopentylamino)purine (Compound 71)

6-chloropurine was combined with R-(+)-α,α-diphenyl-2-pyrrolidinemethanol in n-butanol with two equivalents of N(iPr)₂Et. The reaction was heated to 95 □C and allowed to react for 24 h. Solvent was removed from the reaction mixture *in vacuo* and the crude product purified by silica gel chromatography.

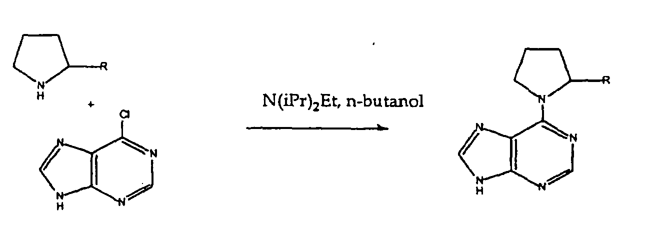

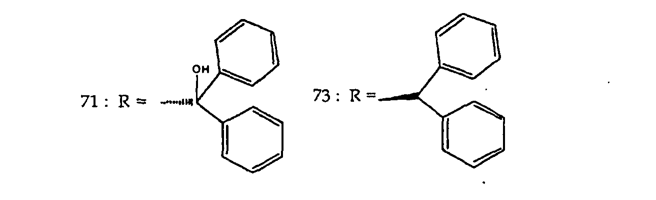

### 3. 2-diphenylpyrrole (Compound 76)

2-pyrrolidinone was combined with dimethylsulphate in benzene and refluxed for 3h. After purification including distillation, the resulting 2-methylimino ester pyrrolidine was combined with excess lithium phenoxide (PhLi) in dry ether at room temperature for 18h to yield the title compound.

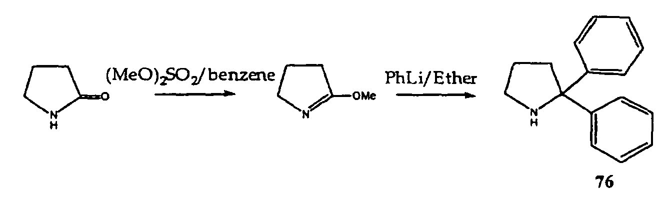

### 4. 6-amino-4(2-diphenylborinic ester) ethylamino pyrimidine (Compound III168)

4-amino-6-hydroxy-2-thiopyrimidine was treated with Raney nickel (RaNi) in water and ammonia and heated to reflux for 2h. Purification afforded the 4-amino-6-hydroxypyrimidine, which was combined with phosphorus oxychloride and N,N-diethylaniline and heated at reflux for 4h to give 4-amino-6-chloropyrimidine. This product was combined with diphenylborinic acid ethanolamine ester in toluene with diisopropylethylamine and heated to reflux overnight to produce the title compound.

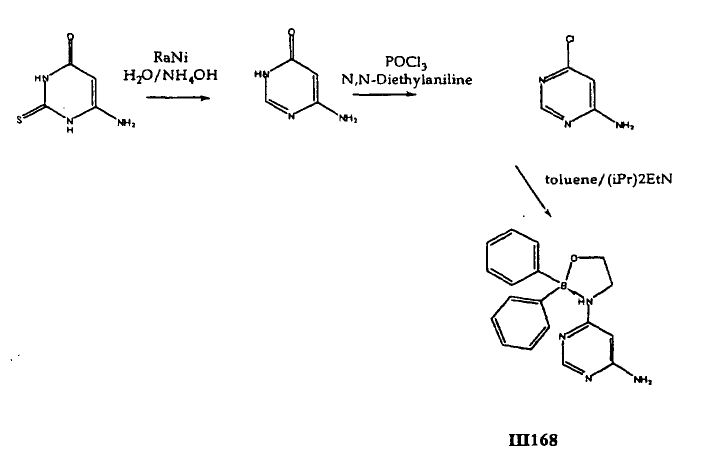

### 5. 4-amino-5(2-diphenylborinic ester ethyliminoester)imidazole (Compound III170)

4-amino-5-imidazolecarboxamide hydrochloride was heated to reflux in phosphorus oxychloride for 3.5hs and after purification gave 4-amino-5-nitrile imidazole. This was resuspended in ethanol saturated with HCl overnight and purification gave 4-amino-5-ethylimino ester imidazole hydrochloride. This was combined with diphenylborinic acid ethanolamine ester in tetrahydrofuran (THF) overnight to yield the title compound.

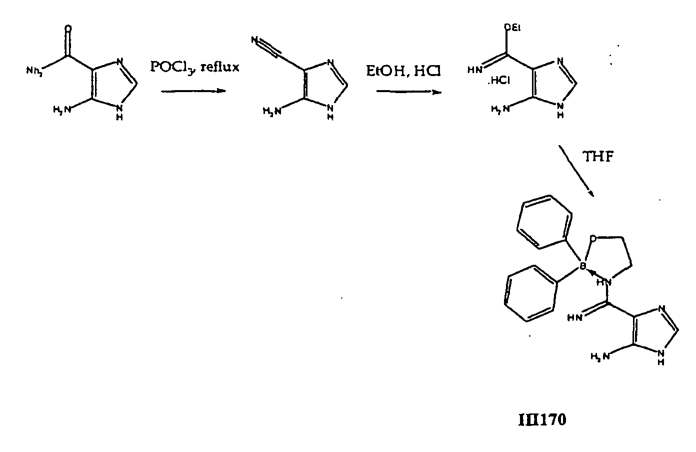

### EXAMPLE 4

### Second Generation Libraries

Two "second generation libraries were prepared based on the compounds:

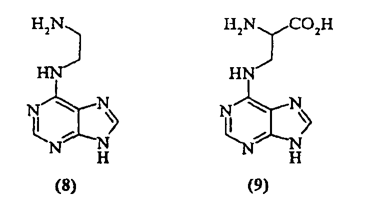

The first second generation library ("library A") was constructed as shown below from parent compound **78**. Compound **78** was combined with one equivalent of aldehyde (or ketone) in methanol at 25□C in a heater-shaker. Reactions were set up in duplicate. After reaction for one hour, BH₃-resin was added and the mixture was allowed to react overnight. To one set of the reaction was added a second equivalent of one of the following aldehydes:
cyclohexanecarboxaldehyde;
3-furaldehyde;
1-methyl-2-pyrrolecarboxaldehyde;
hydrocinnamaldehyde;
4-pyridine carboxaldehyde;
2-phenylproprionaldehyde;
phenylacetaldehyde;
m-anisaldehyde;
heptaldehyde;
3-nitrobenzaldehyde;
3-phenylbutyraldehyde;
3-pyridylacetaldehyde N-oxide;
ethyl leveulinate;
ethyl -2-ethylacetoacetone;
ethyl-4-acetylbutyrate;
ethyl proprionylacetate;
ethyl 2-benzylacetoacetone;
1-phenyl-2-pentanone;
1-carbethoxy-4-piperidone;
N-acetonylphthalimide;
2-fluorophenylacetone;
4-(3-oxobutyl)phenylacetate.

The entire plate was then allowed to react for a further 24 hrs. The compounds generated are either monoalkylated, with R=H, R'= alkyl, aryl or dialkylated with R=R'=alkyl or aryl.

The other second generation library ("library B") was constructed by reacting the parent compound **III142** with the amines used to construct the N6 library and set forth above in the presence of trimethyl aluminum (AlMe₃) in dichloromethane at 50□C overnight. The solvent was removed by evaporation, and the residue was dissolved in acetonitrile and treated with trimethylsilyl iodide overnight. Reactions were worked up by adding methanol, evaporating the solvents, partitioning the residue between ether and water/acetic acid (7:3) and extracting the product into the aqueous layer.

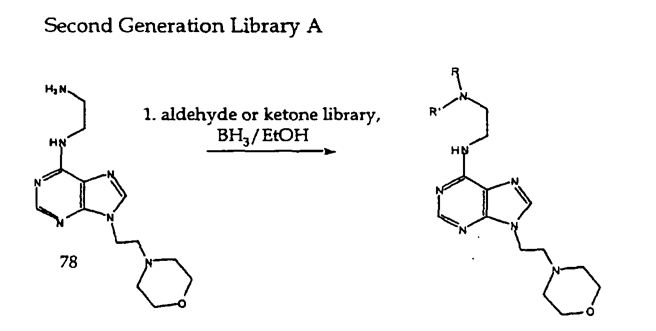

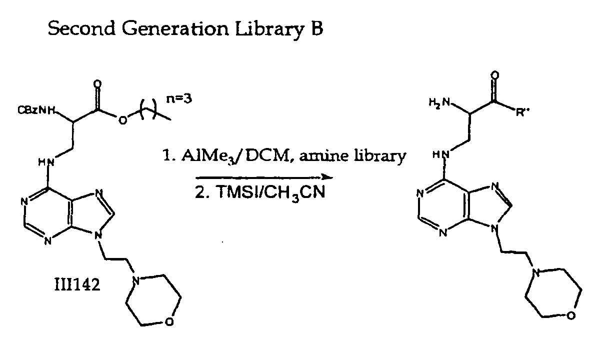

### EXAMPLE 5

### Compounds based on Diphenyl Borinic Esters

Based on the results disclosed above, one common component of several of the adenine DNA methyltransferase inhibitors of the invention is diphenyl borinic ester. Accordingly, several additional compounds based on this ester were prepared as follows. These compounds have the following structures: 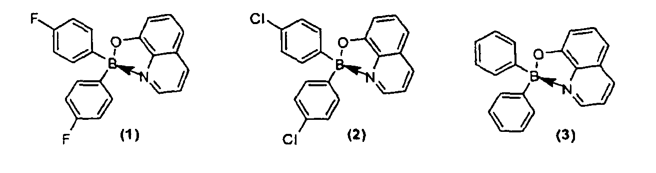 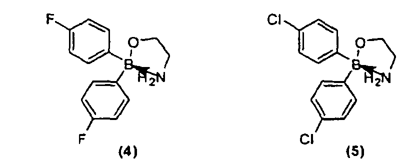 The general synthesis of these compounds is shown in Reaction Scheme 7.

### 1. Synthesis of borinic acids (Compound 8).

Dichloroborane dimethyl sulfide complex (0.5 - 2mL) was dissolved in either tetrahydrofuran or diethyl ether under argon and cooled to -78°C. he appropriate phenyl Grignard reagent (2 molar equivalents), in tetrahydrofuran, diethyl ether, cyclohexane or mixtures of these solvents, was added dropwise to the cold reaction. The reaction was allowed to warm to room temperature and stirred overnight. Diethyl ether was added to the reaction and the reaction was hydrolyzed by the slow addition of 1N hydrochloric acid. The layers were separated and the organic layer was washed with saturated aqueous NaCl. The organic layer was dried over magnesium sulfate (MgSO₄), filtered and the solvent was removed *in vacuo* to give the crude product as a clear oil. This crude preparation of the title compound was used directly in the next stage of the synthesis 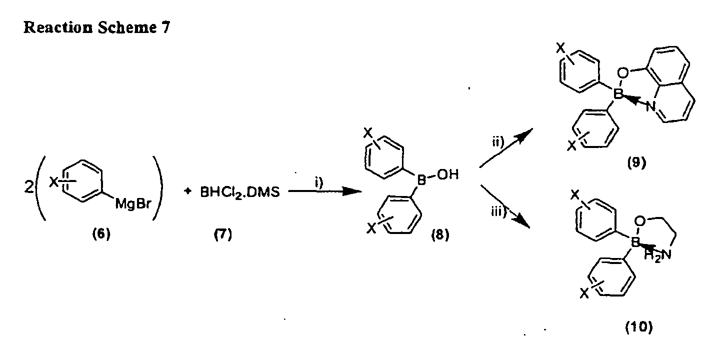 where the reaction conditions are:
i) tetrahydrofuran (THF) or ethyl ether (Et₂O), -78 °C to room temperature overnight;
ii) EtOH, 8-hydroxyquinoline, room temperature;
iii) EtOH, 2-aminoethanol, room temperature.
X can represent up to 5 substituents on each phenyl group, which can be independently hydrogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from oxygen and nitrogen, and where any member of the alkyl, aryl or cycloalkyl group is optionally substituted with halogen, lower alkyl or lower alkoxy, aryl or substituted aryl, halide, nitro, nitroso, aldehyde, carboxylic acid, esters, amides, or sulfates.

### 2. Synthesis of borinic acid 8-hydroxyquinoline esters (Compounds 9).

The crude borinic acid (**8**) was dissolved in 0.05-5mL ethanol and was treated with 1-2 equivalents of 1M 8-hydroxyquinoline in ethanol. The product either precipitated from the solution or the solution was concentrated and left to crystallize, once a solid had formed; the product was collected by filtration and washed with ethanol.

### 3. Synthesis of borinic acid ethanolamine esters (Compounds 10).

The crude borinic acid (**8**) was dissolved in 0.05-5mL ethanol and treated with 1-2 equivalents of 1M ethanolamine in ethanol. The product either precipitated from the solution or the solution was concentrated and left to crystallize; once a solid had formed, the product was collected by filtration and washed with ethanol.

The compounds (**1**) - (**5**) prepared as described above were tested using the *in vivo* assays of the invention using *Caulobacter cresentus,* and compounds (**1**), (**2**), (**4**) and (**5**) have been tested for cell growth inhibition against *Bacillus subtilis.* The IC₅₀ values are shown in Table II.

**TABLE II**

| | Compound (1) | Compound (2) | Compound (3) | Compound (4) | Compound (5) |
|---|---|---|---|---|---|
| *Caulobacter cresentus* | 23 µM | 16 µM | >100 µM | 85 µM | 17 µM |
| *Bacillus subtili*s | 14 µM | 7 µM | Not tested | 34 µM | 32 µM |

These compounds have advantageous physical properties, and are isolated as pure, stable solids that are amenable to large-scale production. Additional specific embodiments of adenine DNA methyltransferase inhibitors of the invention includes related compounds having these additional features:
1) Analogues with various substituents on the phenyl rings in any, or combination of, the ortho-, meta- and para- positions, including fused rings and substituted fused rings;
2) Analogues having aromatic heterocycles of various ring sizes, substituted heterocycles, fused heterocycles and substituted fused heterocycles in place of one or both phenyl groups;
3) Analogues having two non-identical aromatic rings bound to the boron atom, using combinations of the aromatic systems described in 1) and 2) above;
4) Analogues prepared using quinolines (**9**) containing various substituents in any possible position or structural analogues including fused heteroaromatic rings containing one or more heteroatom in any possible position or fused heteroaromatic rings containing one or more heteroatom in any possible position and containing various substituents in any possible position; and
5) Analogues having substitutions on either, or both of, the C-1 and C-2 positions of the ethylene group of the 2-aminoethanol of (**10**).

It should be understood that the foregoing disclosure emphasizes certain specific embodiments of the invention and that all modifications or alternatives equivalent thereto are within the spirit and scope of the invention as set forth in the appended claims.

## Claims

1. A compound of the formula 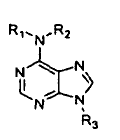 or a pharmaceutically acceptable salt thereof,
wherein R', R², and R³ are the same or different and are independently hydrogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from oxygen and nitrogen, and where any member of the alkyl, aryl or cycloalkyl group is optionally substituted with halogen, lower alkyl or lower alkoxy, aryl or substituted aryl, and
where R³ can be ribose, deoxyribose or phosphorylated derivatives thereof,
wherein R¹, R², and R³ are not all hydrogen and
wherein when R³ is ribose, deoxyribose or phosphorylated derivatives thereof, one of R¹ or R² is not hydrogen.

2. A compound according to claim 1, wherein R¹ and R² are the same or different and are independently hydrogen, histamine dihydrochloride, norphenylephrine hydrochloride, 1,2-diaminopropane, 5-amino-1,3,3-trimethylcyclohexanemethyl-amine, 3-isopropoxypropylamine, diphenylborinic acid, ethanolamine ester, 2-(2-aminoethylamino)-ethanol, tetrahydrofurfurylamine, 5-methyltryptamine hydrochloride, 3,3-diphenylpropylamine, 1-(3-aminopropyl)-2-pyrrolidinone, 2-(2-aminoethyl)- 1-methylpyrrolidine, 2-(aminomethyl)benzimidazole dihydro-chloridehyrate, 2,2,2-trifluoroethylamine, hydrochloride, L-camosine, (R)-(-)-1 -amino-2-propanot, 2-(1-cyclohexenyl)ethylamine, 4-(trifluoromethyl)benzylamine, 2,5-dichloroamylamine hydrochloride, (+/-)-4-amino-3-bydroxybutyric acid, N,N-dimethylethylenediamine, 3,3-dimethylbutylamine, 1,4-diamino-2-butanone dihydrochloride, aminomethylbenzoic acid, aminohydroxymethylpropane diol, 2-(aminoethyl)pyridine, aminobutanol, adamantamine, aminohexanoic acid, N-benyzylethanolamine, ethyl-6-aminobutyrate, hydrochloride, ethylenediamine, or 2-cyclohex-1-enylethylamine.

3. A compound according to claim 1, wherein R³ is methyl 4-iodobutyrate, 1-bromo-3-phenylpropane, cinnamyl bromide, 2-chloroethylphosphonic acid, ethyl 2-(2-chloroacetamido)-4-thiazole-acctate, 4-(2-chloroethyl)morpholine hydrochloride, (2-bromoethyl)trimethylammonium bromide, 4-chlorophenyl 2-bromoethyl ether, N-(3-bromopropyl)pthalimide, 2-chloroethyl, isocyanate, 2-chloro-N, N-dimethylacetoacetamide, 3-chloro-2-hydroxypropyl methacrylate, 2-bromo-2'-hydroxy-5'-nitroacetanilide, 3-(2-bromoethyl)indole, 5-chloro-2-pentanone ethylene ketal, 2-chloroethyl ethyl sulfide, 3-chloro-N-hydroxy-2,2-dimethyl-propionamide, L-1-p-Tosylamino-2-Phenylethyl chloromethyl ketone, 2-(2-bromoethyl)- 1,3-dioxolane, ethyl 2-(bromomethyl)acrylate, 2-(2-(2-chlorethyoxy)ethoxy)ethanol, (3-chloropropyl)trimethoxysilane, chloramphenicol, 4-(chloromethyl)benzoic acid, bromoethylamine hydrochloride, epibromohydrin, iodopentane, or benzyl bromide

4. A compound according to claim 1, wherein R¹ is H, R² is (2-diphenylborinic ester) ethyl or diphenylpropyl, and R³ is H, 2-(4-morpholinyl)-ethyl, 3-(N-phthaloyl)-aminopropyl, 2-(2-(2-hydroxyethoxy)ethoxy)ethy), or ethyl-2-(acrylate)-methyl.

5. A compound according to claim 1, wherein R¹ is H, R² is (S-homocysteinyl)methyl and R³ is ribose, 5'phosphorylribose, deoxyribose or 5' phosphoryl deoxyribose.

6. A compound according to claim 1, wherein R³ is H and R¹ and R² are together 2-(diphenylmethyl) cyclopentyl or 2-(diphenylhydroxymethyl) cyclopentyl.

7. A compound according to claim 1, wherein R¹ is H, R² is alanylbutyl ester, 2-alkylketone-2-aminoethyl, 2-aminoethyl or mono- or bisubstituted 2-amino ethyl, and R³ is 2-(4-morpholinyl)-ethyl.

8. A compound according to claim 1, wherein R¹ and R² are the same or different and are independently hydrogen, histamine dihydrochloride, norphenylephrine hydrochloride, 1,2-diaminopropane, 5-amino-1,3,3-trimethylcyclohexanemethyl-amine, 3-isopropoxypropylamine, diphenylborinic acid, ethanolamine ester, 2-(2-aminoethylamino)-ethanol, tetrahydrofurfurylamine, 5-methyltryptamine hydrochloride, 3,3-diphenylpropylamine, 1-(3-aminopropyl)-2-pyrrolidinone, 2-(2-aminoethyl)-1-methylpyrrolidine, 2-(aminomethyl)benzimidazole dihydro-chloridehyrate, 2,2,2-trifluoroethylamine, hydrochloride, L-carnosine, (R)-(-)-1-amino-2-propanol, 2-(1-cyclohexenyl)ethylamine, 4-(trifluoromethyl)benzylamine, 2,5-dichloroamylamine hydrochloride, (+/-)4-amino-3-hydroxybutyric acid, N,N-dimethylethylenediamine, 3,3-dimethylbutylamine, 1,4-diamino-2-butanone dihydrochloride, aminomethylbenzoic acid, aminohydroxymethylpropane diol, 2-(aminoethyl)pyridine, aminobutanol, adamantamine, aminohexanoic acid, N-benyzylethanolarnine, ethyl-6-aminobutyrate, hydrochloride, ethylenediamine, or 2-cyclohex-1-enylethylamine, and R³ is methyl 4-iodobutyrate, 1-bromo-3-phenylpropane, cinnamyl bromide, 2-chloroethylphosphonic acid, ethyl 2-(2-chloroacetamido)-4-thiazole-acetate, 4-(2-chloroethyl)morpholine hydrochloride, (2-bromoethyl)trimethylammonium bromide, 4-chlorophenyl 2-bromoethyl ether, N-(3-bromopropyl)pthalimide, 2-chloroethyl, isocyanate, 2-chloro-N, N-dimethylacetoacetamide, 3-chloro-2-hydroxypropyl methacrylate, 2-bromo-2'-hydroxy-5'-nitroacetanilide, 3-(2-bromoethyl)indole, 5-chloro-2-pentanone ethylene ketal, 2-chloroethyl ethyl sulfide, 3-chloro-N-hydroxy-2,2-dimethyl-propionamide, L-1-p-Tosylamino-2-Phenylethyl chloromethyl ketone, 2-(2-bromoethyl)-1,3-dioxolane. ethyl 2-(bromomethyl)acrylate, 2-(2-(2-chlorethyoxy)ethoxy)ethanol, (3-chloropropyl)trimethoxysilane, chloramphenicol, 4-(chloromethyl)benzoic acid, bromoethylamine hydrochloride, epibromohydrin, iodopentane, or benzyl bromide.

9. A compound according to claim 1, selected from 6-N-(diphenylborinic ester)-ethyl-adenine, 6-N-(diphenylborinic ester)-ethyl-9-(2-(4-morpholinyl)-ethyl)-adenine, 6-N-(diphenylborinic ester)-ethyl-9-(3-(N-phthaloyl)-aminopropyl)-adenine, 6-N-(diphenylborinic ester)-ethyl-9-(2-(2-(2-hydroxyethoxy)ethoxy)ethyl)-adenine, and 6-N-(diphenylborinic ester)-ethyl-9-(ethyl-2-acrylate)-methyl-adenine.

10. A pharmaceutical composition comprising a compound according to Claim 1 combined with at least one pharmaceutically acceptable carrier or excipient.

11. A method for the treatment of a disease or disorder associated with infection with a pathogenic bacteria that expresses an adenine DNA methyltransferase, said method comprising administering to a patient in need of such treatment a therapeutically-cffective amount of a compound of claim 1.

12. A method according to Claim 11 wherein the disease or disorder associated infection with a pathogenic bacteria is *Staphylococcus aureus; Staphylococcus saprophyticus; Streptococcus pyrogenes; Streptococcus agalactiae; Streptococcus pneumoniae; Bacillus anthracis; Corynebacterium diphtheria; Clostridium perfringens; Clostridium botulinum; Clostridium tetani; Neisseria gonorrhoeae; Neisseria meningitidis; Pseudomonas aeruginosa; Legionella pneumophila; Escherichia coli; Yersinia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus* or *Agrobacterium tumefaciens.*

13. A compound of the formula 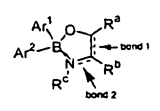 or a pharmaceutically acceptable salt thereof,
wherein R^{a}, R^{b}, and R^{c} are the same or different and are independently hydrogen, halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, or wherein R^{a}, R^{b} and R^{c} may be connected by aromatic, aliphatic, heteroaromatic, heteroaliphatic ring structures or substituted embodiments thereof, and
wherein Ar¹ and Ar² can be the same or different and are each independently aryl or aryl substituted at one or a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, and
wherein bond 1 and bond 2 are independently a single bond or a double bond.

14. A compound according to claim 14, selected from di-(p-fluorophenyl)borinic acid 8- hydroxyquiniline ester, di-(p-chlorophenyl)borinic acid 8-hydroxyquiniline ester, diphenylborinic acid 8-hydroxyquiniline ester, di-(p-fluorophenyl)borinic acid ethanolamine ester, and di-(p-chlorophenyl)borinic acid ethanolamine ester.

15. A pharmaceutical composition comprising a compound according to Claim 1 combined with at least one pharmaceutically acceptable carrier or excipient.

16. A method for the treatment of a disease or disorder associated with infection with a pathogenic bacteria that expresses an adenine DNA methyltransferase, said method comprising administering to a patient in need of such treatment a therapeutically-effective amount of a compound of claim 1.

17. A method according to Claim 11 wherein the disease or disorder associated infection with a pathogenic bacteria is *Staphylococcus aureus; Staphylococcus saprophyticus; Streptococcus pyrogenes; Streptococcus agalactiae; Streptococcus pneumoniae; Bacillus anthracis; Corynebacterium diphtheria; Clostridium perfringens; Clostridium botulinum; Clostridium tetani; Neisseria gonorrhoeae; Neisseria* *meningitidis; Pseudomonas aeruginosa; Legionella pneumophila; Escherichia coli; Yersinia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus* or *Agrobacterium tumefaciens.*

18. A combinatorial library comprising a multiplicity of compounds according to claim1.

19. A combinatorial library comprising a multiplicity of compounds according to claim 13.

20. A packaged pharmaceutical composition comprising the pharmaceutical composition of Claim 10 in a container and instructions for using the composition to treat a patient suffering from a disease or disorder associated with infection with a pathogenic bacteria.
